# EUROPEAN PATENT SPECIFICATION

(11) **EP 2 814 425 B1**
(45) Date of publication and mention of the grant of the patent: **13.02.2019**
(21) Application number: 13748720.3
(22) Date of filing: 14.02.2013
(51) Int. Cl.: A61F 2/07, A61F 2/06, A61F 2/82, A61L 27/14

(54) **KINK RESISTANT GRAFT DEVICES**
KNICKBESTÄNDIGE TRANSPLANTATVORRICHTUNGEN
DISPOSITIFS DE GREFFE RÉSISTANTS AU VRILLAGE

(30) Priority: 14.02.2012 US 201261598705 P
(43) Date of publication of application: 24.12.2014
(73) Proprietor: Neograft Technologies, Inc., Taunton, MA 02780 (US)
(72) Inventor: SOLETTI, Lorenzo, Pittsburgh, PA 15218 (US); EL-KURDI, Mohammed S., Mansfield, MA 02048 (US); FLAHERTY, J. Christopher, Auburndale, FL 33823 (US); MCGRATH, Jon, Duxbury, MA 02332 (US)
(74) Representative: Barker Brettell LLP
(86) International application number: PCT/US2013/026079
(87) International publication number: WO 2013/123147

(56) References cited:
- WO-A1-01/35863
- WO-A1-2007/003199
- WO-A1-2007/003199
- WO-A1-2010/058406
- WO-A1-2011/146733
- WO-A2-2005/065578
- WO-A2-2005/074547
- WO-A2-2006/026725
- WO-A2-2006/099016
- WO-A2-2009/002827
- WO-A2-2011/084559
- US-A- 4 323 525
- US-A- 4 798 606
- US-A- 5 723 004
- US-A1- 2005 154 446
- US-A1- 2006 257 447
- US-A1- 2007 027 529
- US-A1- 2011 137 404
- US-A1- 2011 288 628

## Description

### RELATED APPLICATIONS

This application claims benefit of U.S. Provisional Patent Application Serial Number 61/598,705, filed February 14, 2012. This application is further related to U.S. Patent Application Serial Number 12/022,430, filed January 30, 2008; U.S. Patent Application Serial Number 13/515,996, filed June 14, 2012; and International Patent Application Serial Number PCT/US2012/21209, filed January 13, 2012.

### TECHNICAL FIELD

The present invention relates to graft devices which are constructed and arranged to resist kinking.

### BACKGROUND

Coronary artery disease, leading to myocardial infarction and ischemia, is a leading cause of morbidity and mortality worldwide. Current treatment alternatives include percutaneous transluminal angioplasty, stenting, and coronary artery bypass grafting (CABG). CABG can be carried out using either arterial or venous conduits and is an effective and widely used treatment to combat coronary arterial stenosis, with nearly 500,000 procedures being performed annually. In addition, there are approximately 80,000 lower extremity bypass surgeries performed annually. The venous conduit used for bypass procedures is typically the autogenous saphenous vein and remains the graft of choice for 95% of surgeons performing these bypass procedures. According to the American Heart Association, in 2004 there were 427,000 bypass procedures performed in 249,000 patients. The long term outcome of these procedures is limited due to occlusion of the graft vein or anastomotic site as a result of intimal hyperplasia (IH), which can occur over a timeframe of months to years.

Development of successful small diameter synthetic or tissue engineered vascular grafts has yet to be accomplished and use of arterial grafts (internal mammary, radial, or gastroepiploic arteries, for example) is limited by the short size, small diameter and availability of these veins. Despite their wide use, failure of arterial vein grafts (AVGs) remains a major problem: 12% to 27% of AVGs become occluded in the first year with a subsequent annual occlusive rate of 2% to 4%. Patients with failed AVGs usually require clinical intervention such as an additional surgery.

IH accounts for 20% to 40% of all AVG failures within the first 5 years after CABG surgery. Several studies have determined that IH develops, to some extent, in all mature AVGs and this development is regarded by many as an unavoidable response of the vein to grafting. IH is characterized by phenotypic modulation, followed by de-adhesion and migration of medial and adventitial smooth muscle cells (SMCs) and myofibroblasts into the intima where they proliferate. In many cases, this response can lead to stenosis and diminished blood flow through the graft. It is thought that IH may be initiated by the abrupt exposure of the veins to the dynamic mechanical environment of the arterial circulation.

WO2005/065578 describes a medical device for implantation in a vessel, and, more particularly, a vascular prosthesis with anastomotic device. The medical device comprises at least one anastomotic member, at least partially interposing a non-woven liner of electrospun fibers and a non-woven cover of electrospun fibers. The at least one anastomotic member is designed for engaging at least one end of the medical device to a wall of the vessel upon implantation of the medical device within the vessel.

WO2011/084559 discloses a tubular graft device comprising a tubular member and a fiber matrix of one or more polymers about a circumference of the tubular member. The matrix may be electrospun onto the tubular tissue. In one example, the tubular tissue is from a vein, such as a harvested saphenous vein, useful as an arterial graft, for example and without limitation, in a coronary artery bypass procedure. Also provided is method of preparing a tubular graft and connecting the graft between a first body space and a second body space, such as the aorta and a location on an occluded coronary artery, distal to the occlusion.

WO2009/002827 discloses a stent-graft including a substrate, a helix disposed about the substrate, a covering disposed over at least a portion of the helix, and a support member coupled to the covering.

WO01/35863 discloses a modular elongated stent having an overlap region where two modular components fit together, the overlap region being relatively stiff as compared to another more flexible region of the stent when the stent is in an assembled configuration, the stent further comprising a mimic region that has a stiffness essentially equivalent to the stiffness of the overlap region, to provide kink resistance. A stent having such a mimic region or otherwise stiff region and a flexible region may have a transition region between the stiff and flexible regions, such as a bridging material attached to the stent, also to provide kink resistance. A stent may have relatively stiff regions and relatively flexible regions positioned to align the flexible regions with curved regions of a body lumen when deployed within the body lumen. The stiffness of the stiff, flexible, and transition regions may be controlled by attaching material to the stent, varying the cross-sectional area of the stent components, varying the metallurgical properties thereof, and/or by varying the stent architecture. Methods for providing kink resistance by controlling stent stiffness are also disclosed. A stent having regions of different metallurgical properties is also disclosed, as are methods for creating such a stent.

WO2010/058406 discloses an external vein support comprising an elongate axial body including an axis, said body being plastically deformable by at least one of stretching, bending, twisting, and any combination thereof, relative to said axis.

### SUMMARY

For at least the reasons discussed above and for other reasons, there is a need for systems, methods, and devices which provide enhanced AVGs and other improved grafts for mammalian patients. Desirably, such systems, methods, and devices are expected to improve long term patency and minimize surgical and device complications such as those caused by kinking of graft devices.

Insofar as the terms "invention" and/or "embodiment" are used in the following, and/or features are presented as being optional, this should be interpreted in such a way that the only protection sought is that of the invention as claimed.

According to one aspect of the technology disclosed and claimed herein, there is provided a graft device for a mammalian patient as defined in claim 1. The graft device is configured to limit (e.g., prevent) luminal narrowing and/or radial collapse of the graft device.

The kink resisting element can provide one or more advantages selected from the group consisting of: minimizing undesirable conditions, such as buckling, conduit deformation, luminal deformation, stasis, flows characterized by significant secondary components of velocity vectors such as vortical, recirculating or turbulent flows, luminal collapse, and/or thrombus formation; preserving laminar flow such as preserving laminar flow with minimal secondary components of velocity, such as blood flow through the graft device, blood flow proximal to the graft device and/or blood flow distal to the graft device; limiting (e.g., preventing) bending and/or allowing proper bending of the graft device, such as bending that occurs during and/or after the implantation procedure; limiting (e.g., preventing) accumulation of debris; limiting (e.g., preventing) stress concentration on the tubular wall; and combinations of these advantages.

The kink resisting element can be positioned at a location selected from the group consisting of: inside the tubular conduit; between the tubular conduit and the fiber matrix; within a wall of the fiber matrix; outside the fiber matrix; and combinations of these.

The kink resisting element can comprise a durable material. Additionally or alternatively, the kink resisting element can include at least a portion comprising a biodegradable material, for example Magnesium. The kink resisting element can comprise a material, e.g. a biodegradable material or other material configured to provide temporary kink resistance, for example for a duration of up to twenty four hours, up to one month, or up to six months.

The fiber matrix comprises the kink resisting element. In one embodiment, a portion of the fiber matrix is modified to provide kink resistance where the modified parameter is selected from the group consisting of: stiffness; hardness; thickness; materials of construction; and combinations of these. For example, the kink resisting element can comprise at least a portion of the fiber matrix which has been hardened, softened, melted, stiffened, modified to include a resilient bias, expanded and/or contracted. In some embodiments, the modified portion of the fiber matrix includes a resiliently biased structure. In some embodiments, the kink resisting element comprises a recess in the fiber matrix where the recess depth is less than or approximates the fiber matrix thickness proximate the recess. Alternatively or additionally, the recess width can be less than, equal to, or greater than half of the fiber matrix circumference. In some embodiments, the kink resisting element comprises multiple recesses in the fiber matrix. For example, the fiber matrix can comprise a partial circumferential portion extending along a relatively straight line along the length of the fiber matrix, and the recesses are positioned in the partial circumferential portion. Alternatively, the fiber matrix can comprise a first partial circumferential portion extending along a first relatively straight line along the length of the fiber matrix and a second partial circumferential portion extending along a different second relatively straight line along the length of the fiber matrix and wherein a first recess is positioned in the first partial circumferential portion and a second recess is positioned in the second partial circumferential portion. The fiber matrix can comprise a curved bias as a result of the fiber matrix being applied to the tubular conduit while the tubular conduit is placed on a curved mandrel. Alternatively, the fiber matrix curved bias can be created after the fiber matrix is applied to the tubular conduit.

The tubular conduit can comprise the kink resisting element. For example, the kink resisting element can comprise at least a portion of the tubular conduit which has been hardened, softened, melted, stiffened, modified to include a resilient bias, expanded and/or contracted. The kink resisting element can comprise a component or multiple components separate from the fiber matrix selected from the group consisting of: a helical coil; a sleeve; an adhesive; an exoskeletal structure; rings; rings and connecting elements; radial projections; and combinations of these. The kink resisting element can be inserted within the conduit, between the conduit and the fiber matrix and/or external to the fiber matrix. The kink resisting element can comprise a material selected from the group consisting of: a metal or combinations of metals such as a shape memory material (e.g. Nitinol); another alloy of titanium; titanium; stainless steel; a cobalt-chrome-molybdenum alloy (e.g. MP35N and/or Elgiloy); a polymer or combination of polymers such as silicone; Pebax; polypropylene; nylon; PTFE; polyethylene; UWMWPE; polyurethane; polycarbonate; and combinations of these.

The kink resisting element can comprise at least one coil. The coil diameter can approximate the fiber matrix diameter. The coil can comprise a material selected from the group consisting of: a metal or combinations of metals such as a shape memory material (e.g. Nitinol); another alloy of titanium; titanium; stainless steel; a cobalt-chrome-molybdenum alloy (e.g. MP35N and/or Elgiloy); a polymer or combination of polymers such as silicone; Pebax; polypropylene; nylon; PTFE; polyethylene; UWMWPE; polyurethane; polycarbonate; and combinations of these. In some embodiments, the fiber matrix comprises the coil.

The kink resisting element can comprise an adhesive where the adhesive is selected from the group consisting of: fibrin glue; polyurethane; polyacrylamide; polyimide; epoxy; silicone; cyanoacrylate; polyacrylate; polyacrylamide; polyethylene oxide; poloxymer; polysaccharides; polymerized proteins; carbohydrates; glycoproteins; mucopolysaccharides; polyphenolic proteins; reactive adhesives; non-reactive adhesives; blood; and combinations of these. The adhesive can comprise blood, e.g. the patient's blood. The adhesive can be positioned in multiple discrete locations, or the adhesive can be applied, arranged, disposed and/or deposited in a pattern. The adhesive can be a temporary adhesive, for example adhesion can cease in less than twenty four hours, less than one week, less than one month, or less than six months.

As noted above, the kink resisting element can comprise an exoskeletal structure comprising one or more collars, a plurality of scales, or a combination thereof. The length of the exoskeletal structure can be less that the graft device length. The exoskeletal structure can be configured to provide a single degree of freedom and lock in multiple directions. Alternatively, the exoskeletal structure can be configured to provide multiple degrees of freedom and lock in a single direction.

The kink resisting element can comprise one or more collars. The fiber matrix can comprise one or more collars. The collars can be distributed along a portion of the graft device. In some embodiments, the collars are not distributed on at least one end portion of the graft device.

The kink resisting element can comprise two or more rings and intervening connectors. The fiber matrix can comprise the two or more rings and the intervening connectors. In one embodiment, the intervening connectors comprise chain-like structures. The chains can comprise a material selected from the group consisting of: a shape memory material, such as Nitinol; other titanium alloys; stainless steel; cobalt-chrome-molybdenum alloys, silicone, Pebax, polypropylene, nylon, PTFE, UWMWPE, or other polymers; and combinations of these. Alternatively, the chain-like structures can comprise a mesh. The rings can comprise a material selected from the group consisting of: a shape memory material, such as Nitinol; other titanium alloys; stainless steel; cobalt-chrome-molybdenum alloys, silicone, Pebax, polypropylene, nylon, PTFE, UWMWPE, or other polymers; and combinations of these. Alternatively or additionally, the rings can comprise a magnetic material, for example a first ring can be configured to magnetically attract and/or repel a second ring. In some embodiments, the kink resisting element can comprise an accordion structure.

The kink resisting element can comprise one or more radial projections. The fiber matrix can comprise a circumferential portion where one or more radial projections comprise a ring projection that extends 360° about said circumferential portion. Alternatively, the ring projection extends less than 360°, for example 180° or 90°about said circumferential portion. The one or more radial projections can comprise a projection with a length less than or equal to 20% of the fiber matrix diameter.

The tubular conduit can comprise a varying circumferential shape, and the fiber matrix can conform to the varying circumferential shape of the tubular conduit. The tubular conduit can comprise a harvested tissue such as tissue selected from the group consisting of: vein such as a saphenous vein; artery; urethra; intestine; esophagus; ureter; trachea; bronchi; duct; fallopian tube; and combinations of these. Alternatively or additionally, the tubular conduit can comprise an artificial material such as a material selected from the group consisting of: polytetrafluoroethylene (PTFE); expanded PTFE (ePTFE); polyester; polyvinylidene fluoride / hexafluoropropylene (PVDF-HFP); silicone; polyethylene; polypropylene; polyester based polymer; polyether based polymer; thermoplastic rubber; and combinations of these.

The fiber matrix can comprise at least one polymer, for example a polymer selected from the group consisting of; polyolefins; polyurethanes; polyvinylchlorides; polyamides; polyimides; polyacrylates; polyphenolics; polystyrene; polycaprolactone; polylactic acid; polyglycolic acid; and combinations of these. In some embodiments, the matrix comprises a polymer applied in combination with a solvent where the solvent is selected from the group consisting of: hexafluoroisopropanol; acetone; methyl ethyl ketone; benzene; toluene; xylene; dimethyleformamide; dimethylacetamide; propanol; ethanol; methanol; propylene glycol; ethylene glycol; trichloroethane; trichloroethylene; carbon tetrachloride; tetrahydrofuran; cyclohexone; cyclohexpropylene glycol; DMSO; tetrahydrofuran; chloroform; methylene chloride; and combinations of these. The fiber matrix can comprise a thermoplastic co-polymer comprising two or more materials, for example a first material and a softer second material. The fiber matrix can comprise a durable and/or a biodegradable material. The fiber matrix can comprise an electrospun fiber matrix.

The fiber matrix can comprise a fiber matrix applied with a device selected from the group consisting of: an electrospinning device; a melt-spinning device; a melt-electrospinning device; a misting assembly; a sprayer; an electrosprayer; and combinations of these.

According to another aspect of the technology, which is described but not claimed, a graft device for a mammalian patient comprises a tubular conduit; a conformal covering surrounding the tubular member; and a kink resisting element.

According to another aspect of the technology, which is described but not claimed, a system for creating a graft device comprises a tubular conduit and a fiber matrix delivery assembly configured to deliver a fiber matrix to surround the tubular conduit, where the graft device is constructed and arranged to be kink resistant, for example via a kink resisting element.

The system can further comprise a placement device configured to place the kink resisting element. In some embodiments, the fiber matrix delivery assembly comprises the placement device. The kink resisting element can comprise an element selected from the group consisting of: an adhesive; one or more rings such as two or more rings connected with a chain or mesh; a coil such as a helical coil; an accordion structure; an exoskeletal structure; one or more radial projections; and combinations of these. The kink resisting element can be applied prior to delivery of the fiber matrix, during delivery of the fiber matrix and/or after delivery of the fiber matrix.

The system can further comprise a fiber matrix modifying assembly configured to modify the fiber matrix to provide kink resistance. In some embodiments, the fiber matrix delivery assembly comprises the fiber matrix modifying assembly. In some embodiments, the fiber matrix modifying assembly can comprise a handheld component. The kink resisting element can comprise a portion of the fiber matrix modified by the fiber matrix modifying assembly. For example, the modified portion of the fiber matrix can be a thickened portion and/or a portion comprising a modified shape. In some embodiments, the kink resisting element can comprise one or more slots in the fiber matrix, said slots created by the fiber matrix modifying assembly. Similarly, the fiber matrix modifying assembly can create on ore more of: a recess; a ring; a coil; a radial projection; and combinations of these. The fiber matrix modifying assembly can be configured to be translated and/or rotated about the tubular conduit. The fiber matrix modifying assembly can be selected from the group consisting of: a nozzle; an energy delivery element such as a laser energy delivery element; a fluid jet; a cutting element; a mechanical abrader; and combinations of these. The fiber matrix modifying assembly can modify the fiber matrix during and/or after said application of the fiber matrix. Additionally, the fiber matrix modifying assembly can be configured to deliver an agent.

The fiber matrix modifying assembly can comprise an aperture positioned between the fiber matrix delivery assembly and the tubular conduit.

The fiber matrix modifying assembly can comprise an electromagnetic field modifying element constructed and arranged to modify the flight path of fibers delivered to the fiber matrix by the fiber matrix delivery assembly.

The fiber matrix delivery assembly can comprise a device selected from the group consisting of: an electrospinning device; a melt-spinning device; a melt-electrospinning device; a misting assembly; a sprayer; an electrosprayer; and combinations of these.

The system can further comprise a patterning mask, e.g. a chemical mask, positioned on the tubular conduit and/or the fiber matrix. The patterning mask can be biodegradable. The patterning mask can be removable.

The system can further comprise a curved mandrel configured to reside within the tubular conduit as the fiber matrix is applied.

The system can be constructed and arranged to apply an adhesive configured to provide kink resistance to the graft device.

According to another aspect of the technology, which is described but not claimed, a method for producing a graft device for a mammalian patient comprises providing a tubular conduit and applying a fiber matrix surrounding the tubular conduit, where the graft device is configured to be kink resistant.

The method can further comprise providing a kink resisting element. In some embodiments, the kink resisting element comprises a separate component from the fiber matrix, for example, the kink resisting element is positioned at a location selected from the group consisting of: inside the tubular conduit; between the tubular conduit and the fiber matrix; within a wall of the fiber matrix; outside the fiber matrix; and combinations of these. In some embodiments, the application of the fiber matrix causes the graft device to be kink resistant, for example, the fiber matrix comprises the kink resisting element.

The method can further comprise applying a kink resisting element. The kink resisting element can be applied at a time selected from the group consisting of: prior to application of the fiber matrix; during application of the fiber matrix; after application of the fiber matrix; and combinations of these. The method can further comprise implanting the tubular conduit and fiber matrix into a patient, wherein the applying of the kink resisting element is performed after said implantation. The kink resisting element can comprise a coil such that the application of the kink resisting element comprises unwrapping and/or unwinding the coil.

The method can further comprise inserting the tubular conduit onto a curved mandrel where the application of the fiber matrix is performed after inserting the tubular conduit onto the curved mandrel.

### BRIEF DESCRIPTION OF THE DRAWINGS

**Fig. 1** is a schematic illustration of an example graft device including a kink resisting element comprising a helical coil, according to an illustrative embodiment of the technology described herein, which is described but not claimed.
**Fig. 2** is a schematic illustration of an example graft device including a kink resisting element positioned on an external surface of a fiber matrix, according to an illustrative embodiment of the technology described herein, which is described but not claimed.
**Fig. 3** is a schematic illustration of an example graft device including a fiber matrix created and/or modified to provide kink resistance, according to an illustrative embodiment of the technology described herein, which is described but not claimed.
**Figs. 3A through 3E** are schematic illustrations of five example graft devices, respectively, each including a fiber matrix created and/or modified to provide kink resistance, according to illustrative embodiments of the technology described herein, which is described but not claimed.
**Figs. 4A and 4B** are sequential, schematic illustrations depicting two steps, respectively, of an example process for making a graft device including a kink resisting element, according to an illustrative embodiment of the technology described herein, which is described but not claimed.
**Fig. 5** is a schematic illustration of an example graft device including multiple adhered portions configured to provide kink resistance, according to an illustrative embodiment of the technology described herein, which is described but not claimed.
**Fig. 6** is a schematic illustration of an example graft device including a multi-segmented exoskeletal structure configured to provide kink resistance, according to an illustrative embodiment of the technology described and claimed herein.
**Fig. 7** is a schematic illustration of an example graft device including multiple sliding circumferential collars configured to provide kink resistance, according to an illustrative embodiment of the technology described and claimed herein.
**Figs. 8A through 8C** are schematic illustrations of three example graft devices, respectively, each including multiple rings with intervening connecting segments that are configured to provide kink resistance, according to an illustrative embodiment of the technology described herein, which is described but not claimed.
**Fig. 9** is a schematic illustration of an example graft device including a fiber matrix in a curved orientation that is configured to provide kink resistance, according to an illustrative embodiment of the technology described herein, which is described but not claimed.
**Fig. 10** is a flow chart depicting a method for creating a graft device configured to resist kinking, according to an illustrative embodiment of the technology described herein, which is described but not claimed.
**Fig. 11** is a schematic illustration of an example system for producing a kink resistant graft device, according to an illustrative embodiment of the technology described herein, which is described but not claimed.

### DETAILED DESCRIPTION

Reference will now be made in detail to example embodiments of the devices, systems, and methods described herein, some of which are illustrated in the accompanying drawings. The same reference numbers are used throughout the drawings to refer to the same, like, or similar components (e.g., parts).

Provided herein are graft devices for implantation in a mammalian patient. The graft devices include a conduit, such as harvested tissue such as a harvested vein graft, and a fiber matrix that surrounds the conduit. The fiber matrix is typically applied with an electrospinning device, a melt-spinning device, a melt-electrospinning device, a misting assembly, a sprayer, an electrosprayer, a reservoir configured for a dipping process, or other fiber matrix delivery device.

The graft devices further include a kink resisting element, such as to limit (e.g., prevent) luminal narrowing, radial collapse and/or other kinking of the graft device, such as during implantation of the graft device during a surgical procedure and/or at a time after implantation. The kink resisting element typically provides one or more advantages selected from the group consisting of: minimizing undesirable conditions, such as buckling, conduit deformation, luminal deformation, stasis, flows characterized by significant secondary components of velocity vectors such as vortical, recirculating or turbulent flows, luminal collapse, and/or thrombus formation; preserving laminar flow such as preserving laminar flow with minimal secondary components of velocity, such as blood flow through the graft device, blood flow proximal to the graft device and/or blood flow distal to the graft device; limiting (e.g., preventing) bending and/or allowing proper bending of the graft device, such as bending that occurs during and/or after the implantation procedure; limiting (e.g., preventing) accumulation of debris; limiting (e.g., preventing) stress concentration on the tubular wall; and combinations of these. The kink resisting element can be permanent or temporary, and can include portions configured to provide permanent and/or temporary kink resistance to all or portions of the graft device.

The kink resisting element can be the fiber matrix itself, or one or more portions of the fiber matrix modified or otherwise configured to resist kinking. The fiber matrix can include varying properties along its circumference and/or its length that are configured to provide kink resistance. Alternatively or additionally, the kink resisting element can comprise a separate component such as a spring or other element configured to resist kinking. The kink resisting element can be placed inside the conduit, between the conduit and the fiber matrix, between layers or within layers of the fiber matrix and/or outside the fiber matrix. The kink resisting element can comprise a biodegradable or bioerodible (hereinafter "biodegradable") material or otherwise be configured to provide a temporary kink resistance to the graft device.

Also provided herein are systems and methods for creating a graft device comprising a conduit, a surrounding fiber matrix and a kink resisting element. Systems typically include an electrospinning unit and/or other fiber delivering assembly. In some embodiment, the kink resisting element comprises a component that is applied and/or inserted, such as with the fiber matrix delivery assembly or with a placement or insertion device. Alternatively or additionally, the kink resisting element can be created by modifying the fiber matrix, such as with the fiber matrix delivery assembly or other fiber modifying device.

The devices described herein can include electrospun fiber matrices, such as those disclosed in U.S. Application Serial No. 13/502,759. The inventive concepts described herein can also include graft devices, as well as systems, tools and methods for producing graft devices, such as those disclosed in applicant's co-pending PCT applications: Serial No. PCT/US12/21209; Serial No. 13/811,206; and Serial No. PCT/US12/24251.

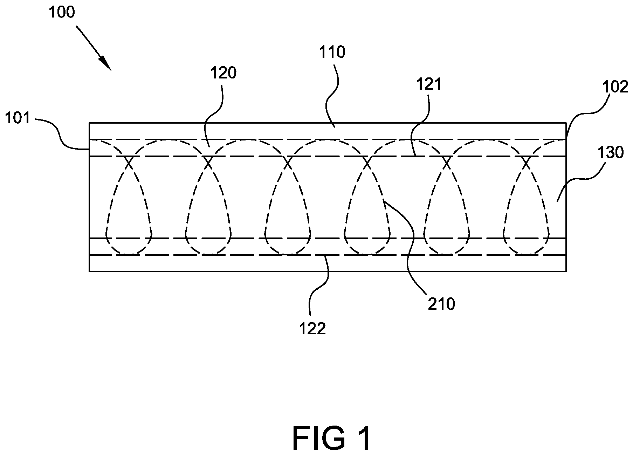

**Referring now to** **Fig. 1**, a side view of a graft device including a kink resisting element comprising a helical coil, is illustrated. Graft device 100 includes tubular conduit 120, having inner wall 121 and outer wall 122, circumferentially surrounded by a covering, fiber matrix 110. Graft device 100 includes a first end 101 and a second end 102, and is typically configured to be placed between a first body location and a second body location of a patient. Graft device 100 includes lumen 130 from first end 101 to second end 102, such as to carry blood or other fluid when graft device 100 is connected between two vessels, such as two blood vessels.

Graft device 100 further includes a kink resisting element, coil 210. Coil 210 is constructed and arranged to limit (e.g., prevent) graft device 100 from kinking, such as during implantation procedure and/or while under stresses endured during its functional lifespan. In some embodiments, coil 210 surrounds conduit 120, between outer wall 122 and fiber matrix 110, as is shown in Fig. 1 where coil 210 comprises a diameter approximating the outer diameter of conduit 120. In some embodiments, coil 210, in whole or in part, can be within fiber matrix 110 and/or surrounding fiber matrix 110. In some embodiments, multiple coils 210 can exist, each surrounding conduit 120, surrounding fiber matrix 110 and/or positioned within fiber matrix 110.

Coil 210 can comprise a material selected from the group consisting of: a metal or combinations of metals such as a shape memory material (e.g. Nitinol); another alloy of titanium; titanium; stainless steel; a cobalt-chrome-molybdenum alloy (e.g. MP35N and/or Elgiloy); a polymer or combination of polymers such as silicone; Pebax; polypropylene; nylon; PTFE; polyethylene; UWMWPE; polyurethane; polycarbonate; and combinations of these.

In some embodiments, coil 210 or a portion of coil 210 can comprise a biodegradable material, such as a biodegradable metal or polymer. Coil 210 can be configured to biodegrade over time such as to provide a temporary kink resistance to device 100. In some embodiments, coil 210 can temporarily provide kink resistance to graft device 100 for a period of less than 24 hours. In some embodiments, coil 210 can provide kink resistance to graft device 100 for a period of less than one month. In some embodiments, coil 210 can provide kink resistance to graft device 100 for a period of less than six months. Numerous forms of biodegradable materials can be employed. Bolz et al (U.S. Patent Serial No.: 09/339,927) discloses a bioabsorbable implant which includes a combination of metal materials that can be an alloy or a local galvanic element. Metal alloys consisting of at least a first component which forms a protecting passivation coat and a second component to ensure sufficient corrosion of the alloy. The first component is at least one component selected from the group consisting of: magnesium, titanium, zirconium, niobium, tantalum, zinc and silicon, and the second component is at least one metal selected from the group consisting of: lithium, sodium, potassium, manganese, calcium and iron. Furst et al (U.S. Patent Application Serial No.: 11/368,298) discloses an implantable device at least partially formed of a bioabsorbable metal alloy that includes a majority weight percent of magnesium and at least one metal selected from calcium, a rare earth metal, yttrium, zinc and/or zirconium. Doty et al (U.S. Patent Application Serial No.: 11/744,977) discloses a bioabsorbable magnesium reinforced polymer stent that includes magnesium or magnesium alloys. Numerous biodegradable polymers can be used such as: polylactide, poylglycolide, polysaccharides, proteins, polyesters, polyhydroxyal kanoates, polyalkelene esters, polyamides, polycaprolactone, polyvinyl esters, polyamide esters, polyvinyl alcohols, polyanhydrides and their copolymers, modified derivatives of caprolactone polymers, polytrimethylene carbonate, polyacrylates, polyethylene glycol, hydrogels, photo-curable hydrogels, terminal diols, and combinations thereof. Dunn et al (U.S. Patent Serial No.: 06/563,191) discloses a medical implant including bioabsorbable fibers that reinforce a bioabsorbable polymer matrix.

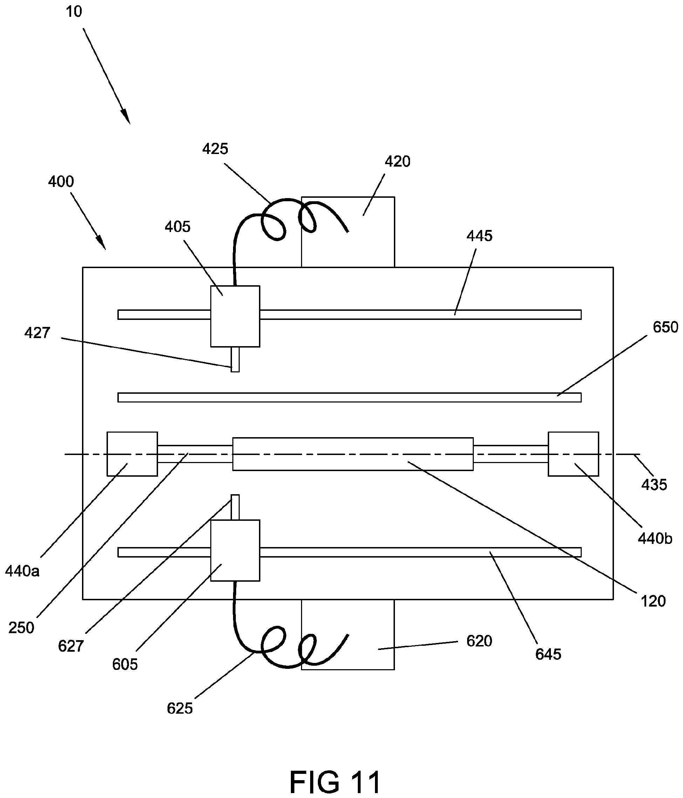

In some embodiments, coil 210 comprises multiple fibers applied in a helical coil configuration, such as multiple fibers applied prior to, during and/or after application of fiber matrix 110. Coil 210 can comprise similar or dissimilar materials to fiber matrix 110, and can be applied with similar or dissimilar devices, such as fiber applied by an electrospinning unit, such as is described in detail herein in reference to Fig. 11. In some embodiments, coil 210 can comprise one or more sections of fiber matrix 110, such as one or more helical sections of fiber matrix 110, which have properties different that the remaining portions of fiber matrix 110, such as sections that have different stiffness, hardness, thickness and/or materials of construction, such as sections that are configured to provide a structural bias or resilience, similar to a helical spring. A fiber modifying device, such as that described in reference to Fig. 11, can be used to modify the one or more sections of fiber matrix 110.

Conduit 120 can include a portion having a varying cross-sectional shape, such that a fiber matrix 110 deposited on conduit 120 conforms to the varying circumferential shape over the portion of conduit 120. In some embodiments, conduit 120 comprises a harvested vessel, such as a harvested vein, and the varying cross-sectional shape includes one or more of: a longitudinal segment including a sidebranch such as a ligated sidebranch; a longitudinal segment that includes a varying non-circular geometry; a longitudinal segment that includes a tapered inner and/or outer diameter; and combinations of these. Fiber matrix 110 can have varying thicknesses, such as to accommodate a varying cross-sectional shape of conduit 120.

Conduit 120 can include living tissue, for example, a vessel harvested from a mammalian patient. The conduit comprising living tissue can be selected from the group consisting of: vein, such as a saphenous vein; artery; urethra; intestine; esophagus; ureter; trachea; bronchi; duct; fallopian tube; and combinations of these. Alternatively or additionally, conduit 120 can be an artificial conduit such as a conduit constructed of materials selected from the group consisting of: polytetrafluoroethylene (PTFE); expanded PTFE (ePTFE); polyester; polyvinylidene fluoride / hexafluoropropylene (PVDF-HFP); silicone; polyethylene; polypropylene; polyester based polymer; polyether based polymer; thermoplastic rubber; and combinations of these.

Typically, graft device 100 includes a restrictive matrix including polymers such as biodegradable polymers and/or non-biodegradable (i.e. durable) polymers. Typically, the polymer solution includes one or more polymers and one or more solvents, such as polymers configured to be electrospun about conduit 120, as is described in reference to Fig. 11. Polymers can be selected from the group consisting of: polyolefins; polyurethanes; polyvinylchlorides; polyamides; polyimides; polyacrylates; polyphenolics; polystyrene; polycaprolactone; polylactic acid; polyglycolic acid; and combinations of these. Solvents can be selected from the group consisting of: hexafluoroisopropanol; acetone; methyl ethyl ketone; benzene; toluene; xylene; dimethyleformamide; dimethylacetamide; propanol; ethanol; methanol; propylene glycol; ethylene glycol; trichloroethane; trichloroethylene; carbon tetrachloride; tetrahydrofuran; cyclohexone; cyclohexpropylene glycol; DMSO; tetrahydrofuran; chloroform; methylene chloride; and combinations of these.

The fiber matrix can comprise a thermoplastic co-polymer made of two or more materials, such as a first material and a harder second material. In some embodiments, the co-polymer has a durometer of approximately 55D, with approximately an even amount of the softer material and the harder material. The softer material can include polydimethylsiloxane (PDMS) and a polyether-based polyurethane. The harder material can include aromatic methylene diphenyl isocyanate (MDI).

Graft device 100 can include a first portion, such as a first layer, that is biodegradable and a second portion, such as a second layer, that is not biodegradable. Fiber matrix 110 can be hydrophilic and/or contain one or more hydrophilic materials. Fiber matrix 110 can be applied using an electrospinning process, such as the electrospinning process described in detail in reference to Fig. 11. The electrospinning process can be performed in an operating room, such as when conduit 120 is a harvested saphenous vein graft to be anastomosed between the aorta and a location on a diseased coronary artery distal to an occlusion. In these cardiovascular bypass procedures, end to side anastomotic connections are typically used to attach device 100 to the aorta and the diseased artery. Alternatively, a side to side anastomosis can be used, such as to attach an end of device 100 to multiple arteries in a serial fashion.

Fiber matrix 110 can be processed in a way specific to a patient morphological or functional parameter. These parameters can be selected from the group consisting of: vessel size such as diameter, length, and/or wall thickness; taper or other geometric property of a harvested vessel or other vessel intended for anastomotic attachment; size and location of one or more side branch ostium or antrum of the harvested vessel; patient age or sex; vessel elasticity or compliance; vessel vasculitis or other existing pathological condition; vessel impedance; specific genetic factor or trait of the patient; and combinations of these.

Fiber matrix 110, when used for AVGs, can be processed in a way to achieve a certain blood flow rate or shear stress within the treated AVG. In some typical configurations, the shear stress achieved within the arterial vein graft is between 0.2-3 Pa (2-30 dynes/cm2), preferably 1.2-2 Pa (12-20 dynes/cm2). Fiber matrix 110 can be processed such that the oxygen, nutrients, or cellular permeabilities between the extravascular tissues and the abluminal surface of the treated hollow tissue is controlled. Such permeabilities can depend on a factor selected from the group consisting of: polymer or other fiber material chemical or physical property; fiber matrix pore size distribution; fiber matrix porosity; fiber matrix pore interconnectivity; and combinations of these. In a non-limiting example, cellular permeability can be selectively restricted to reduce leukocyte infiltration across the deposited fiber matrix with pore sizes smaller than seven microns and porosities between 50% and 95%. Generally, oxygen, nutrients, and cellular (e.g., endothelial cells, endothelial progenitor cells, etc.) permeability are required to improve the treated hollow tissue *in vivo* remodeling and healing process. To this end, the pore size range is typically between 10 microns and 1000 microns, preferably between 200 microns and 500 microns, and the porosity typically ranges between 50% and 95%, preferably between 60% and 90%. The pores preferably are highly interconnected so that a relatively straight path along the radial direction of fiber matrix 110 can be traced from most of the pores across the total thickness of fiber matrix 110.

Radial constriction of saphenous vein grafts has been achieved with stent devices placed over the vein prior to anastomosing the graft to the targeted vessels. The devices of the present inventive concepts provide numerous advantages over the stent approaches. For example, the devices described herein can have one or more parameters easily customized to a parameter of the harvested vessel and/or another patient parameter. The fiber matrix can be customized to a harvested vessel parameter such as vessel geometry, such as to reduce the vein internal diameter to produce desired flow characteristics. The fiber matrix can be customized to a target vessel parameter (e.g., the aorta and diseased artery), such as to be compatible with target vessel sizes and/or locations. The fiber matrix can be modified to simplify or otherwise improve the anastomotic connections, such as to be reinforced in the portion of the device that is anastomosed (e.g., portion where suture and/or clips pass through) and/or to protrude beyond the length of the conduit and overlap other members connected to the graft device. The devices of the present inventive concepts can be made to a wide array of lengths during the fabrication procedure, without the need for cutting, converse to the cutting of a stent device, which might result in dangerously sharp edges. The fiber matrix is applied to the tubular conduit in a controlled, repeatable manner, by an apparatus such as an electrospinning instrument. The ends of the fiber matrix are atraumatic, avoiding tissue damage at the anastomotic sites. In addition, the fiber matrix of the present inventive concepts can be configured to be easily and atraumatically removable, such as to apply another fiber matrix. Stent devices are applied manually by the clinician and suffer from numerous issues including but not limited to: providing limited sizes; lacking ability to match harvested vessel irregular topography; being susceptible to fatigue and other metallic structure failure modes; causing of tissue trauma by metal structure; requiring significant manipulation such as manipulation causing vessel trauma; having limited reproducibility and accuracy of placement; being difficult to reposition or remove, particularly without damaging the harvested vessel; and combinations of these.

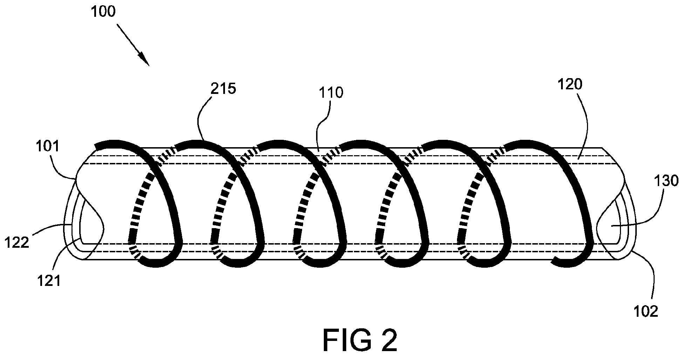

**Referring now to** **Fig. 2****,** a side view of a graft device, including a kink resisting element positioned on the external surface of a fiber matrix, is illustrated. Graft device 100 is of similar construction, and includes components similar to device 100 of Fig. 1. In some embodiments, as illustrated in Fig. 2, device 100 includes a kink resisting element, outer coil 215. Outer coil 215 can comprise a material selected from the group consisting of: a metal or combinations of metals such as a shape memory material (e.g. Nitinol); another alloy of titanium; titanium; stainless steel; a cobalt-chrome-molybdenum alloy (e.g. MP35N and/or Elgiloy); a polymer or combination of polymers such as silicone; Pebax; polypropylene; nylon; PTFE; polyethylene; UWMWPE; polyurethane; polycarbonate; and combinations of these.

In some embodiments, outer coil 215 can comprise a biodegradable material, such as one or more of the biodegradable materials referenced above in reference to coil 210 of Fig. 1, and can be configured to provide kink resistance to graft device 100 for a period of time limited by its biodegradation.

Outer coil 215 can be applied to fiber matrix 110 subsequent to application of fiber matrix 110, such as when coil 215 is applied by a fiber matrix delivery assembly as is described in detail in reference to Fig. 11. Outer coil 215 can be applied to graft device 100 after removal of graft device 100 from a fiber matrix delivery assembly, such as an application by a surgeon before or during a surgical procedure in which device 100 is implanted into a patient. In some embodiments, outer coil 215 can be removed by a surgeon, during or subsequent to the implantation procedure, such as when coil 215 is configured to provide kink resistance for the time period prior to its removal. Application and/or removal of coil 215 can include unwinding coil 215, such as to increase the inner diameter of coil 215 such as to ease insertion of conduit 120 and fiber matrix 110 into coil 215. Removal of coil 215 can include rotating and/or unwrapping coil 215 from around conduit 120 and fiber matrix 110.

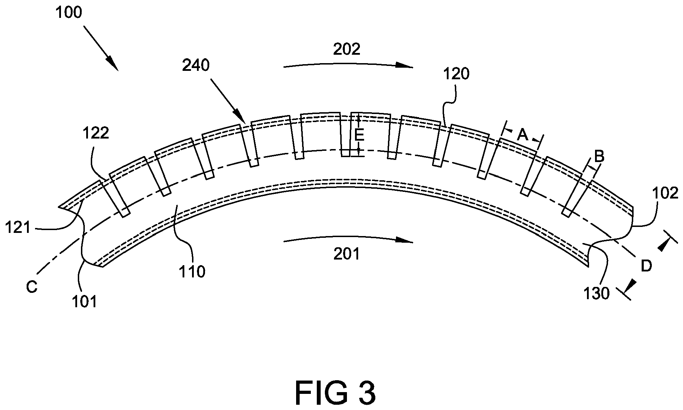

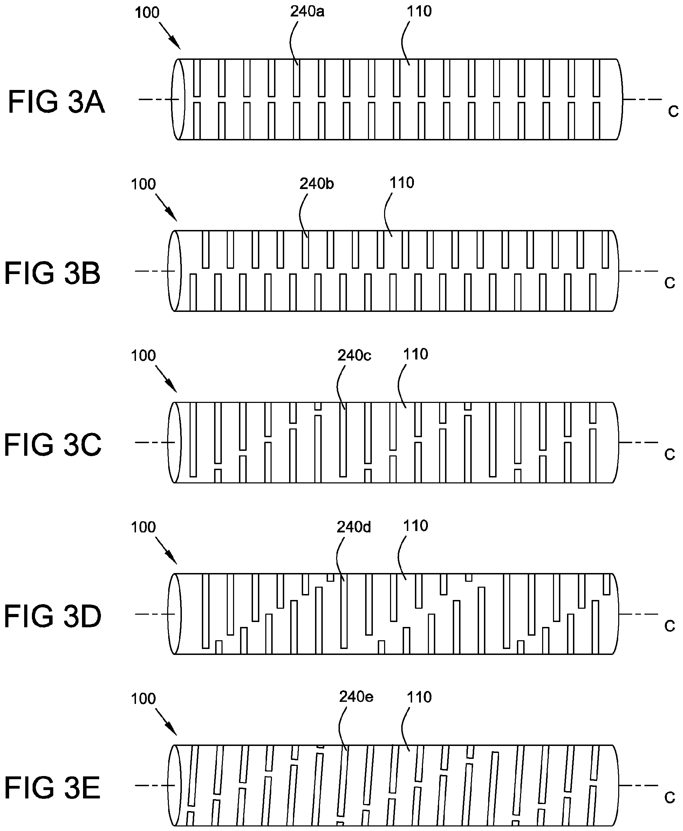

**Referring now to** **Fig. 3****,** a side view of a graft device, including a fiber matrix created and/or modified to provide kink resistance, is illustrated. Graft device 100 includes conduit 120, having inner wall 121 and outer wall 122, circumferentially surrounded by a covering, fiber matrix 110. Graft device 100 is of similar construction, and includes components similar to device 100 of Fig. 1 described hereabove. As illustrated, in some embodiments, fiber matrix 110 comprises relief slots 240, which can extend the full or partial depth of fiber matrix 110. Relief slots 240 and/or other partial or full depth recesses, hereinafter "slots", allow graft device 100 to bend along center line C, such that relief slots 240 align along greater curvature 202, opposite lesser curvature 201, and provide structural strain relief such as to avoid kinking of graft device 100 by increasing the spacing of slots 240 (i.e., expanding) along the greater curvature. Graft device 100 has diameter D and slot depth E. Slot depth E can be chosen to limit (e.g., prevent) kinking under various load or geometric configuration. In some embodiments, slot depth E is greater than 0.5D. In other embodiments, depth E is less than or equal to 0.5D. Graft device 100 has one or more non-slotted portions with length A, with intervening slots with width B. In some embodiments, length B is less than 0.1A. In other embodiments, length B is between 0.1A and 0.3A.

**Figs. 3A through 3E** illustrate sectional views of multiple graft devices, each including a fiber matrix created and/or modified to provide kink resistance. Each graft device 100, including fiber matrix 110, is of similar construction, and includes components similar to device 100 and fiber matrix 110 of Fig. 3. In some embodiments, as illustrated in Fig. 3A, fiber matrix 110 includes multiple longitudinally aligned slots 240a, wherein the slots are relatively orthogonal with central axis C of device 100. In some embodiments, as illustrated in Fig. 3B, fiber matrix 110 includes multiple longitudinally offset slots 240b, wherein the slots are relatively orthogonal with central axis C of device 100. In some embodiments, as illustrated in Fig. 3C, fiber matrix 110 includes multiple longitudinally aligned slots 240c, in the spiral pattern shown, wherein the slots are relatively orthogonal with central axis C of device 100. In some embodiments, as illustrated in Fig. 3D, fiber matrix 110 includes multiple longitudinally offset slots 240d, in the spiral pattern shown, wherein the slots are relatively orthogonal with central axis C of device 100. In some embodiments, as illustrated in Fig. 3E, fiber matrix 110 includes multiple longitudinally aligned slots 240e, wherein the slots are at an acute angle with central axis C of device 100.

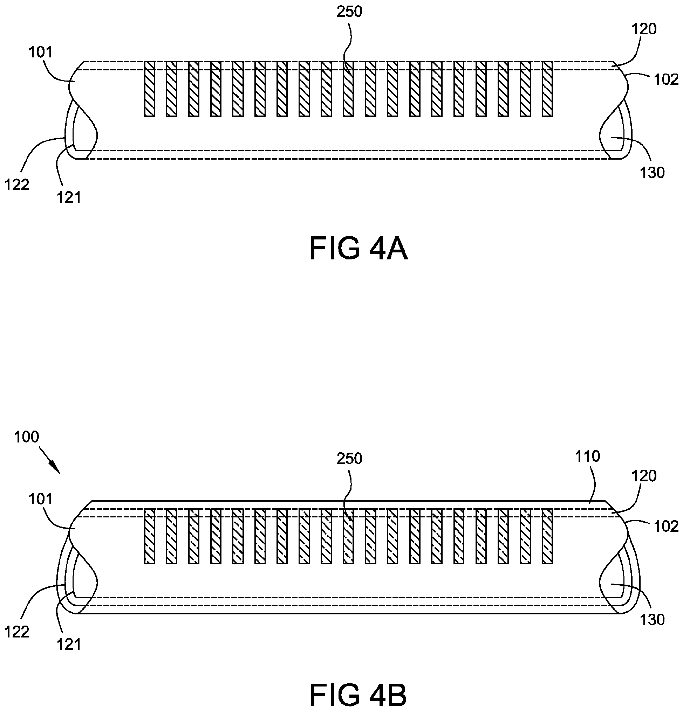

**Referring now to** **Figs. 4A and 4B**, side views illustrating two steps of an example process for making a graft device, including a kink resisting element, are illustrated. **Fig. 4A** shows a conduit 120, such as a harvested saphenous vein segment. Conduit 120 includes inner surface 121 as well as outer surface 122. Multiple segments of adhesive 250 have been applied to outer surface 122 of conduit 120. Adhesive 250 can be applied to conduit 120 by a fiber matrix delivery assembly or other assembly configured to apply material to conduit 120, such as is described in detail in reference to Fig. 11. Adhesive 250 can include a material selected from the group consisting of: fibrin glue; polyurethane; polyacrylamide; polyimide; epoxy; silicone; cyanoacrylate; polyacrylate; polyacrylamide; polyethylene oxide; poloxymer; polysaccharides; polymerized proteins; carbohydrates; glycoproteins; mucopolysaccharides; polyphenolic proteins; reactive adhesives; non-reactive adhesives; and combinations of these. Alternatively or additionally, adhesive 250 can include blood, such as the patient's blood or components of the patient's blood. Alternatively, the blood can include a donor's blood or components of the donor's blood. Use of blood as an adhesive can provide additional benefits, such as improved biocompatibility, reduced irritation, reduced inflammation, and combinations of these. Additionally, adhesive 250 can be a temporary adhesive. Adhesive 250 can be applied to conduit 120 before conduit 120 is inserted into a fiber matrix delivery assembly for the application of a fiber matrix.

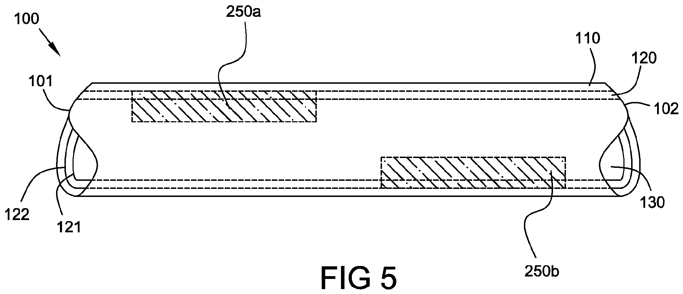

**Fig. 4B** shows graft device 100, comprising conduit 120 of Fig. 4A and further comprising fiber matrix 110, circumferentially surrounding conduit 120 and adhesive 250. Adhesive 250 causes fiber matrix 110 to adhere to outer wall 122 of conduit 120 in the locations in which the segments of adhesive 250 have been applied. In some embodiments, as illustrated in Figs. 4A and 4B, adhesive 250 is applied in a striped pattern, such as to selectively adhere fiber matrix 110 to conduit 120. The selective adherence allows preferential bending of graft device 100, such as to limit (e.g., prevent) kinking of graft device 100 when graft device 100 is placed in a curved configuration and/or is otherwise curved. Adhesive 250 can be applied to one or more portions (such as is shown in Fig. 5), in one or more varied patterns, such as to limit (e.g., prevent) kinking in one or more single or multiple curve orientations.

**Referring now to** **Fig. 5****,** a side view of a graft device, including multiple adhered portions configured to provide kink resistance, is illustrated. Graft device 100 comprises conduit 120 which is surrounded by fiber matrix 110. Fiber matrix 110 is selectively adhered to the outer wall 122 of conduit 120 by two segments of adhesive, adhesive 250a and 250b. In some embodiments, the placement of adhesive 250a and 250b are such that graft device 100 is configured to comprise multiple points of inflection, such as to enable a tortuous path such as when implanted into the chest cavity of a mammalian patient, such as during a cardiovascular bypass procedure. Placement of adhesive 250a and 250b can be such that graft device 100 includes multiple straight sections and one or more points of inflection configured to resist kinking.

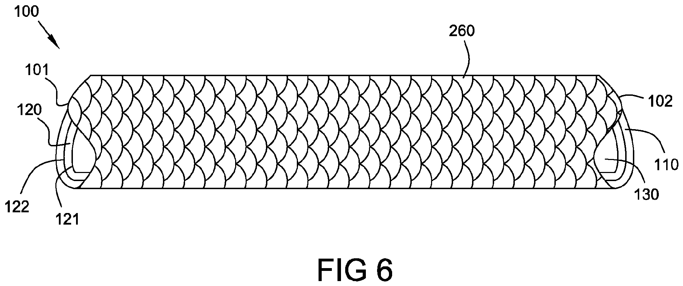

**Referring now to** **Fig. 6****,** a side view of a graft device, including a multi-segmented exoskeletal structure configured to provide kink resistance, is illustrated. Graft device 100 includes conduit 120, having inner wall 121 and outer wall 122. Conduit 120 is circumferentially surrounded by a covering, fiber matrix 110. Graft device 100 is of similar construction, and includes components similar to device 100 of Fig. 1. In some embodiments, as illustrated in Fig. 6, fiber matrix 110 comprises scales 260, constructed and arranged in a fish scale-like construction configured to resist kinking. Scales 260 slidingly engage one another, such as while bending, simultaneously providing flexibility and radial support, such as to limit (e.g., prevent) kinking. In some embodiments, scales 260 are anisotropically linked to each other, such as linking that occurs in the space between scales 260 and the supported vessel. For example, each set of scales 260 can be interlinked such as to resist motion in one direction and allow motion in a different direction. Scales 260 can be constructed and arranged such as to maintain a fixed distance between each set of scales 260 along the circumferential direction of graft device 100 (e.g. to provide circumferential mechanical support), while allowing relative motion, such as sliding, in the longitudinal direction of graft device 100 (e.g. to provide the flexibility needed to allow kink-free flexure and torsion of the graft device).

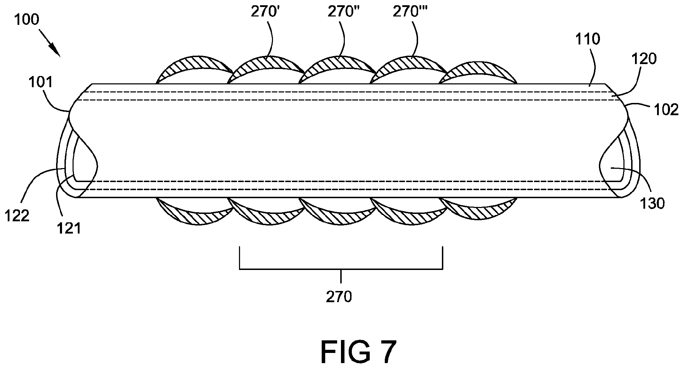

**Referring now to** **Fig. 7****,** a side view of a graft device, including multiple sliding circumferential collars configured to provide kink resistance, is illustrated. Graft device 100, of similar construction and including similar components to device 100 of Fig. 6, comprises conduit 120, surrounded by a fiber matrix 110, further surrounded by collars 270 comprising at least collars 270', 270" and 270"', each collar 270 circumferentially surrounding fiber matrix 110. In some embodiments, fiber matrix 110 comprises the collars 270, such as collars that are distributed during an electrospinning or other fiber applying process. Collar 270" has an outer surface that slidingly engages an inner surface of collar 270', while collar 270" has an inner surface that is slidingly engaged by another surface of collar 270"'. These sliding engagements are constructed and arranged to allow bending of device 100 with multiple degrees of freedom, while providing kink resistance to graft device 100. In some embodiments, collars 270 can surround only a portion of the length of graft device 100, such as a length portion configured to be bent during use. In some embodiments, collars 270 can surround most or all of graft device 100. In some embodiments, collars 270 can surround a middle portion of graft device 100, while ends 101 and 102 of graft device 100 are not covered, such as to avoid interfering with an anastomosis, such as an anastomosis including suture or surgical clips used to create an end-to-side flow connection between each end of device 100 and sides of a first vessel and a second vessel of a patient.

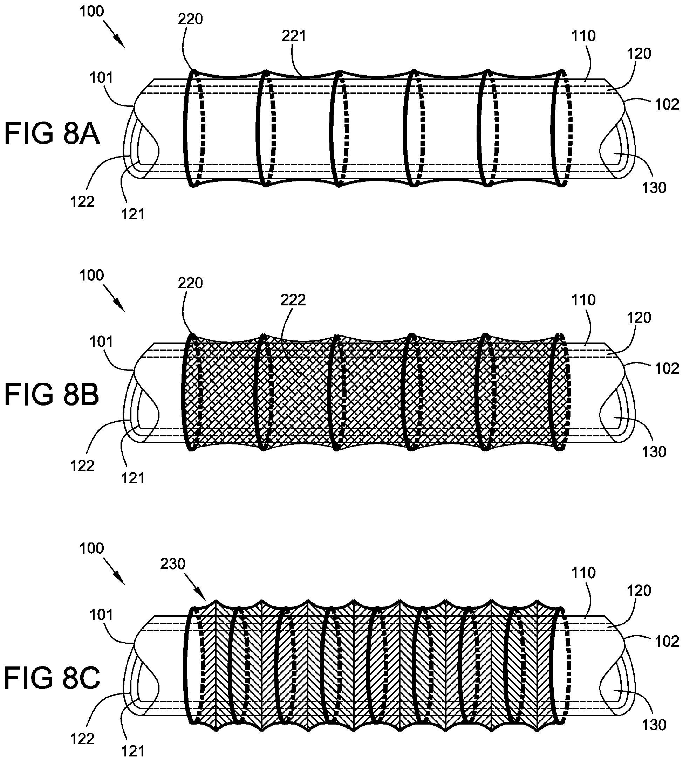

**Referring now to** **Figs. 8A - 8C****,** side views of three graft devices, each including multiple rings with intervening connecting segments and configured to provide kink resistance, are illustrated. Graft devices 100 of Figs. 8A - 8C are of similar construction and including similar components to device 100 of Fig. 1. Each device 100 comprises conduit 120 which is surrounded by a fiber matrix 110, and further surrounded by a kink resisting element. **Referring specifically to** **Fig. 8A****,** the kink resisting element comprises rings 220 and connecting chains 221. The rings 220 and chains 221 construction is configured to provide several degrees of freedom in bending while limiting (e.g., preventing) kinking of graft device 100. Rings 220 can be a material selected from the group consisting of: a shape memory material, such as Nitinol; other titanium alloys; stainless steel; cobalt-chrome-molybdenum alloys, silicone, Pebax, polypropylene, nylon, PTFE, UWMWPE, or other polymers; and combinations of these. In some embodiments, rings 220 can comprise a magnetic material such that magnetic forces between successive rings 220 provide additional kink resistance to graft device 100. Chains 221 provide linear alignment for rings 220, such that rings 220 provide the necessary kink resistance to graft device 100 in the proper location along the length of graft device 100 between first end 101 and second end 102. Chains 221 can be a material selected from the group consisting of: a shape memory material, such as Nitinol; other titanium alloys; stainless steel; cobalt-chrome-molybdenum alloys, silicone, Pebax, polypropylene, nylon, PTFE, UWMWPE, or other polymers; and combinations of these. In some embodiments, chains 221 can be fixedly attached to graft device 100, such as with the use of glue, adhering chains 221 to fiber matrix 110, such as an adhesive described in reference to Fig. 4 hereabove. In some embodiments, fiber matrix 110 comprises chains 221 (e.g. chains 221 are electrospun fibers or modified electrospun fibers), and fiber matrix 110 provides the necessary linear support and alignment to rings 220. Alternatively or additionally, fiber matrix 110 comprises rings 220 (e.g. rings 220 are electrospun fibers or modified electrospun fibers), such as when portions of fiber matrix 110 have a ring-like construction or other varying physical property from other portions of fiber matrix 110.

**Referring now to** **Fig 8B****,** an alternative embodiment of the graft device of Fig. 8A is shown. Graft device 100 of Fig. 8B includes a kink resisting element comprising rings 220 and connecting mesh 222. Mesh 222 is configured to provide similar linear support and alignment to that provided by chains 221 of Fig. 8A, while providing additional circumferential support between rings 220, such as to limit (e.g., prevent) bulging of conduit 120. In some embodiments, fiber matrix 110 can comprise mesh 222 (e.g. mesh 222 comprises electrospun fibers and/or modified electrospun fibers), such that fiber matrix 110 both provides structural support for graft device 100 and linear alignment and positioning to rings 220. Alternatively or additionally, fiber matrix 110 comprises rings 220 (e.g. rings 220 are electrospun fibers or modified electrospun fibers), such as when portions of fiber matrix 110 have a ring-like construction or other varying physical property from other portions of fiber matrix 110. Rings 220 and mesh 222 provide improved kink resistance with minimal effect on axial stiffness.

**Referring now to** **Fig. 8C****,** yet another alternative embodiment of the graft device of Fig. 8A is shown. Graft device 100 includes a kink resisting element comprising a surrounding accordion structure, accordion 230. Accordion 230 is configured to provide bending with several degrees of freedom, while providing kink resistance to graft device 100. The accordion structure can be implemented in such a way as to create one or more stable curved geometries and/or to allow controllable straight or curved elongation, similar to the performance of bendable drinking straws that include an accordion segment.

The kink resisting elements of Figs. 8A - 8C can provide permanent or temporary kink resistance for graft device 100. Rings 220, chains 221, mesh 222, and/or accordion 230 can comprise a biodegradable material, such as a biodegradable material described above in reference to Fig. 1. Temporary kink resistance can provide necessary resistance during an implant procedure, such as a surgical procedure to implant graft device 100 into a patient, while avoiding any undesired long term effects.

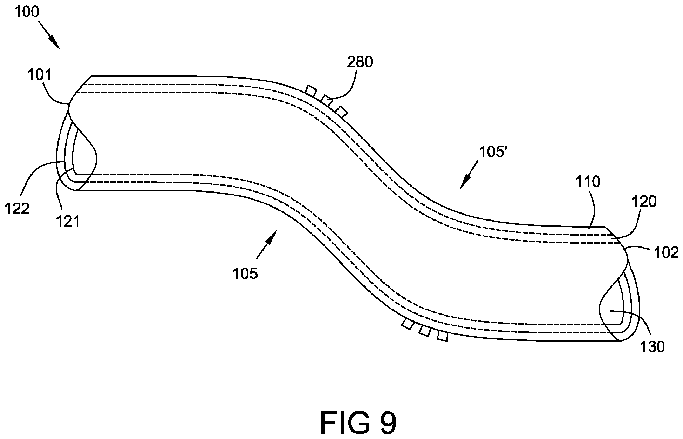

**Referring now to** **Fig. 9****,** a side view of a graft device, including a fiber matrix in a curved orientation and configured to provide kink resistance, is illustrated. Graft device 100 of Fig. 9 is of similar construction and including similar components to device 100 of Fig. 1. Graft device 100 comprises conduit 120, surrounded by fiber matrix 110. Graft device 100 further comprises lumen 130, extending from first end 101 to second end 102. Graft device 100 comprises a graft device with a predetermined bent shape, including bend 105 and bend 105'. Bends 105 and 105' are configured to allow graft device 100 to better match the geometry of the anatomical location chosen for the implantation site of the graft device 100. In some typical embodiments, fiber matrix 110 is applied to conduit 120 while conduit 120 is in the configuration shown, such as when placed on a curved mandrel, not shown but including bends approximating bend 105 a and bend 105b. Application of fiber matrix 110 while conduit 120 is in the curved configuration creates a preferentially curved configuration for device 100, such that the curved geometry of fiber matrix 110 functions as the kink resisting element. In some embodiments, fiber matrix 110 is applied to conduit 120 in a straight configuration, after which a secondary process is performed to elastically or otherwise bias fiber matrix 110 in one or more curved configurations, such as the curved configuration shown in Fig. 9. These preferential biased portions of device 100 resist kinking when device 100 is placed in a patient's anatomy in a configuration similar to the manufactured geometry.

Device 100 can include one or more partial circumferential, radial projections 280, such as one or more partial circumferential projections placed on the outside of curves 105a and/or 105b, such as to provide structural support to further limit (e.g., prevent) kinking, such as to limit (e.g., prevent) radial collapse of fiber matrix 110 and or conduit 120 along the outside of curves 105a and/or 105b. Projections 280 are typically less than one-fifth (i.e. 20%) of the diameter of the fiber matrix 110. In some embodiments, projections 280 are less one-half (i.e. less than 180°) of the circumference of the fiber matrix 110. In other embodiments, projections 280 are less than one-quarter (i.e. less than 90°) of the circumference of the fiber matrix 110.

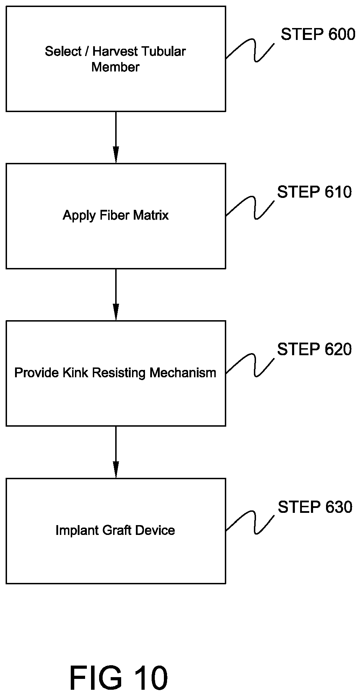

**Referring now to** **Fig. 10****,** a flow chart of a method for creating a graft device configured to resist kinking is illustrated. In STEP 600, a conduit is selected for future implantation into a patient. The conduit can include living and/or artificial tissues, as are described in reference to Fig. 1. In the illustrated method, the conduit includes living tissue, for example, a vessel to be harvested from a mammalian being. In the case of living tissue, the conduit can be selected based upon properties that would be desirable for use in a graft device, which can be subsequently used in a medical procedure, such as a bypass procedure. The particular segment (e.g. length and/or anatomical location) of the conduit to be harvested can also be determined. Subsequent to selecting a conduit, the conduit is harvested, typically from the patient to receive the graft device, such as harvesting via surgical or minimally invasive surgical techniques known to those of skill in the art. Additional procedures and/or treatments can be performed, such as the ligation of one or more side branches of the harvested conduit present along its length.

In STEP 610, a fiber matrix is applied to the harvested conduit, such as via the electrospinning unit described in reference to Fig. 11. The fiber matrix can comprise one or more polymers or other materials as have been described herein.

In STEP 620, a kink resisting element is provided to the harvested conduit. The kink resisting element can comprise a separate component, such as a component selected from the group consisting of: a helical coil; a sleeve; an adhesive; an exoskeletal structure; rings; rings and connecting elements; radial projections; and combinations of these, as has been described herein. The separate component can be placed within the conduit, between the conduit and the fiber matrix and/or external to the fiber matrix. Alternatively or additionally, the providing of the kink resisting element comprises the application of the fiber matrix and/or the modification of the fiber matrix, as has been described herein. Modification of the fiber matrix can include one or more of: creation of slots in the fiber matrix; changing of physical properties of portions of the fiber matrix, such as in one or more patterns; and changing of the shape of the fiber matrix. Alternatively or additionally, the fiber matrix can be applied in a pre-curved shape, such as a geometric configuration anticipated to be encountered after implantation.

In STEP 630, the graft device is implanted in the patient during a medical procedure, for example, during an arterial bypass procedure. Specifically, the graft device can be anastomosed between the aorta and a location on a diseased coronary artery distal to an occlusion. Alternatively, a bypass procedure can be performed in the limb of the patient, such as in the patient's leg. End to side anastomotic connections can be used to attach the graft device to a source of oxygenated blood and a diseased artery. Alternatively, a side to side anastomosis can be used, such as to attach an end of the graft device to multiple arteries in a serial fashion. In other embodiments, the tubular conduit comprises a different form of harvested tissue, or an artificial conduit, and the graft device is placed in one or more body locations, such as to provide a conduit for transfer of one or more fluids or other substances.

Referring now to Fig. 11, a perspective view of an apparatus for producing a kink resistant graft device is illustrated. System 10 includes electrospinning unit 400 and mandrel 250, where conduit 120 has been placed around mandrel 250. Conduit 120 can include living tissue and/or artificial materials, as is described herein. Each end of mandrel 250 is inserted into a rotating drive, motor 440a and 440b, respectively, such that mandrel 250 can be rotated about center axis 435 during application of a fiber matrix, for example fiber matrix 110 described herein. Electrospinning unit 400 can include one or more nozzle assemblies, and in the illustrated embodiment, unit 400 includes nozzle assembly 405, which includes nozzles 427. Nozzle assembly 405 is fluidly attached to polymer solution dispenser 420 via delivery tube 425. Dispenser 420 comprises a solution of one or more polymers. Nozzle assembly 405 is operably attached to a linear drive assembly 445 configured to translate nozzle assembly 405 in at least one direction.

Electrospinning unit 400 can include one or more fiber modification assemblies, and in the illustrated embodiment, unit 400 includes fiber modification assembly 605, which includes fiber modifying element 627. Fiber matrix modification assembly 605 is operably attached to a linear drive assembly 645 configured to translate modification assembly 605 in at least one direction. Fiber modification assembly 605 is operably attached to fiber modification supply assembly 620 via delivery tube 625. Supply assembly 620 can comprise one or more of: a reservoir of one or more agents; a power supply such as a laser power supply; and a reservoir of compressed fluid. In some embodiments, modifying element 627 comprises a nozzle, such as a nozzle configured to deliver a fiber and/or a fiber modifying agent. For clarification, any reference to a "nozzle" and "nozzle assembly" in singular or plural form can include one or more nozzles, such as nozzle 427, and one or more assemblies, such as nozzle assemblies 405.

In some embodiments, fiber modifying element 627 is configured to deliver a kink resisting element, such as an element selected from the group consisting of: an adhesive; one or more rings such as two or more rings connected with a chain or mesh; a coil such as a helical coil; an accordion structure; an exoskeletal structure; one or more radial projections; and combinations of these. Alternatively or additionally, fiber modifying element 627 can be configured to modify conduit 120 and/or fiber matrix 110 to cause a graft device, for example device 100 described herein, to be kink resistant. In these fiber modifying embodiments, fiber modifying element 627 can comprise a component selected from the group consisting of: a nozzle; an energy delivery element such as a laser delivery element such as a laser excimer diode; a fluid jet such as a water jet or air jet; a cutting element; a mechanical abrader; and combinations of these. Modification of fiber matrix 110 can occur during the application of fiber matrix 110 and/or after fiber matrix 110 has been applied to conduit 120.

Fiber modifying element 627 can deliver an agent configured to function as a kink resisting element. In some embodiments, fiber modifying element 627 comprises a nozzle configured to deliver adhesive, such as the adhesive segments configured to provide kink resistance as is described in reference to Fig. 4A hereabove.

Fiber modifying element 627 can comprise a fluid jet, such as a jet configured to deliver a gas or a liquid, such as air or a polymer solvent solution. In these embodiments, fiber modification supply assembly 620 can comprise a compressed air chamber configured to deliver compressed air through delivery tube 625 and modifying element 627, such as compressed air delivered during the application of fiber matrix 110 by nozzle 427.

Fiber modifying element 627 can comprise a laser delivery element, such as a laser diode. For example, in some embodiments, fiber modification supply assembly 620 can comprise a power source and/or an electronic control module configured to power and control the pattern of laser energy delivered by modifying element 627. Laser energy can be delivered during and/or after the application of a fiber matrix 110 to conduit 120, such as to modify fiber matrix 110 to include one or more features selected from the group consisting of: relief slots; rings; coils; radial projections; and combinations of these.

In some (e.g., alternative) embodiments, fiber modification assembly 605 of system 10 can be an additional component, separate from electrospinning unit 400, such as a handheld device configured to deliver a kink resisting element and/or modify fiber matrix 110 and/or conduit 120 such that device 100 is kink resistant. In some embodiments, fiber modification assembly 605 comprises a handheld laser, such as a laser device which can be had operated by an operator. Modification assembly 605 can be used to modify graft device 100 after removal from electrospinning unit 400, such as prior to or during the implantation procedure.

Laser or other modifications to fiber matrix 110 can cause portions of fiber matrix 110 to undergo physical changes, such as hardening, softening, melting, stiffening, creating a resilient bias, expanding, and/or contracting, and/or can also cause fiber matrix 110 to undergo chemical changes, such as forming a chemical bond with an adhesive layer between outer surface 122 of conduit 120 and fiber matrix 110. In some embodiments, fiber modification element 627 is alternatively or additionally configured to modify conduit 120, such that conduit 120 comprises the kink resisting element. Modifications to conduit 120 can include but are not limited to a physical change to one or more portions of conduit 120 selected from the group consisting of: hardening, softening, melting, stiffening, creating a resilient bias, expanding, and/or contracting, and/or can also cause conduit 120 to undergo chemical changes, such as forming a chemical bond with an adhesive layer between an outer surface of conduit 120 and fiber matrix 110.

System 10 can include one or more patterning masks, such as a physical or chemical mask used to limit (e.g., prevent) a fiber matrix from covering portions of conduit 120. For example, in the illustrated embodiment, system 10 includes aperture plate 650. Aperture plate 650 can comprise a stencil-like pattern configured to limit (e.g., prevent) or reduce delivery of fiber from certain areas of outer layer 122 of conduit 120. In some embodiments, aperture plate 650 can comprise a stencil-like pattern that causes fiber matrix 110 to include a pattern of relief slots, such as the relief slots 240 of Fig. 3 hereabove. Alternatively or additionally, aperture plate 650 can be configured to induce one or more changes to the electromagnetic (EM) field within electrospinning unit 400. These one or more changes to the EM field can be configured to cause variations in the delivered fiber pathway, resulting in a patterned fiber matrix, such as the patterned fiber matrix described in detail in reference to Figs. 3A-E hereabove. In some embodiments, mandrel 250 can have modified electrical characteristics, such as modified conductivity along its length, configured to modify the EM field to cause patterned fiber deposition.

In some embodiments, fiber modification assembly 605 can comprise a masking agent delivery assembly. In these embodiments, supply assembly 620 can store a masking solution configured to be delivered through delivery tube 625 to nozzle 627. The masking solution can be delivered to conduit 120 before and/or during the electrospinning of fiber matrix 110. The masking solution can be configured to cause fiber matrix 110 to have a patterned deposition configured as a kink resisting element, such as the patterned fiber matrix described in detail in reference to Figs. 3A-3E hereabove. A mask can be applied to conduit 120 prior to its insertion into electrospinning unit 400, such as by an operator applying a premade covering or sleeve to conduit 120, or by painting or otherwise applying a substance, such as a chemical barrier, to mask conduit 120 from fiber matrix 110. In some embodiments, one or more masks are applied to conduit 120 to modify the EM field used in electrospinning, such as to reduce or limit (e.g., prevent) fiber deposition proximate the mask. The masks can comprise a biodegradable material, such as a material configured to dissolve over time after graft device 100 implantation. The masks can comprise a material which is configured to be washed off of or otherwise be removed from graft device 100 subsequent to the application of fiber matrix 110 to conduit 120.

In some embodiments, the fiber matrix can include an inner layer and an outer layer, where the inner layer can include an adhesive component and/or exhibit adhesive properties. The inner layer can be delivered separate from the outer layer, for example, delivered from a separate nozzle or at a separate time during the process. Selective adhesion between the inner and outer layer can be configured to provide kink resistance.

In some embodiments, electrospinning unit 400 can be configured to deliver the fiber matrix and/or an adhesive layer according to set parameters configured to produce a kink resistant element in and/or provide kink resisting properties to device 100. For example, an adhesive layer can be delivered to conduit 120 for a particular length of time, followed by delivery of a polymer solution for another particular length of time. Other typical application parameters include but are not limited to: amount of adhesive layer and/or polymer solution delivered; rate of adhesive layer and/or polymer solution delivered; nozzle distance to mandrel 250 and/or conduit 120; linear travel distance of a nozzle or a fiber modifying element along its respective drive assembly (for example, drive assembly 445 or 645); linear travel speed of a nozzle or a fiber modifying element along its respective drive assembly; compositions of the polymer solution and/or adhesive layer; concentrations of the polymer solution and/or adhesive layer; solvent compositions and/or concentrations; fiber matrix inner and outer layer compositions and/or concentrations; spontaneous or sequential delivery of the polymer solution and the adhesive layer; voltage applied to the nozzle; voltage applied to the mandrel; viscosity of the polymer solution; temperature of the treatment environment; relative humidity of the treatment environment; airflow within the treatment environment; and combinations of these.

Nozzle 427 and can be constructed of stainless steel. In some embodiments, nozzle 427 has a tubular construction with a length of approximately 3.81 cm (1.5 inches), an ID of approximately 1.1938 mm (0.047 inches) and an OD of approximately 1.651 mm (0.065 inches). Nozzle 427 can include an insulating coating, with the tip of nozzle 427 exposed (e.g. non-insulated), such as with an exposed length of approximately 1cm. Nozzle geometry and electrical potential voltages applied between nozzle 427 and mandrel 250 are chosen to control fiber generation. In some typical embodiments, fibers are created with a diameter between 0.1 µm and 2.0 µm, more typically with a diameter between 0.1 µm and 1.0 µm.

Mandrel 250 is positioned in a particular spaced relationship from nozzle assembly 405 and/or fiber modification assembly 605, and nozzle 427 and/or modifying element 627, respectively. In the illustrated embodiment, mandrel 250 is positioned above and below assemblies 605 and 405, respectively. Alternatively, mandrel 250 can be positioned either above, below, to the right and/or or to the left of, assembly 405 and/or assembly 605. The distance between mandrel 250 and the tip of nozzle 427 and/or modifying element 627 is can be less than 20cm, for example less than 15cm. For example, the tip of nozzle 427 and/or modifying element 627 can be approximately 12.5cm from mandrel 250. In some embodiments, multiple nozzles 427 and/or multiple modifying elements 627, for example components of similar or dissimilar configurations, can be positioned in various orientations relative to mandrel 250. In some embodiments, the distance between nozzles 427 and/or modifying elements 627 varies along the length of mandrel 250, such as to create a varying pattern of fiber matrix 110 along conduit 120. For example, nozzle 427 and/or element 627 distances can vary continuously during the electrospinning process or can vary for a set period of time during the process.

Typically, in some embodiments, an electrical potential is applied between nozzle 427 and one or both of conduit 120 and mandrel 250. The electrical potential can draw at least one fiber from nozzle assembly 405 to conduit 120. Conduit 120 can act as the substrate for the electrospinning process, collecting the fibers that are drawn from nozzle assembly 405 by the electrical potential. In some embodiments, mandrel 250 and/or conduit 120 has a lower voltage than nozzle 427 to create the desired electrical potential. For example, the voltage of mandrel 250 and/or conduit 120 can be a negative or zero voltage while the voltage of nozzle 427 can be a positive voltage. Mandrel 250 and/or conduit 120 can have a voltage of about -5 kV (e.g., - 10kV, -9kV, -8kV, -7kV, -6kV, -5 kV, -4.5 kV-4 kV, -3.5 kV, -3.0kV, -2.5 kV, -2 kV, -1.5 kV, - 1kV) and the nozzle 405 can have a voltage of about +15 kV (e.g., 2.5 kV, 5 kV, 7.5 kV, 12 kV, 13.5 kV, 15 kV, 20kV). In some embodiments, the potential difference between nozzle 427 and mandrel 250 and/or conduit 120 can be from about 5 kV to about 30 kV. This potential difference draws fibers from nozzle 427 to conduit 120. In a preferred embodiment, nozzle 427 is placed at a potential of +15kV while mandrel 250 is placed at a potential of -5kV. In some embodiments, mandrel 250 is a fluid mandrel, such as the fluid mandrel described in applicant's co-pending PCT Application Serial Number PCT/US2011/66905 filed on December 22, 2011.

In some embodiments, a polymer solution, stored in polymer solution dispenser 420, is delivered to nozzle assembly 405 through polymer solution delivery tube 425. The electrical potential between nozzle 427 and conduit 120 and/or mandrel 250 can draw the polymer solution through nozzle 427 of nozzle assembly 405. Electrostatic repulsion, caused by the fluid becoming charged from the electrical potential, counteracts the surface tension of a stream of the polymer solution at nozzle 427 of the nozzle assembly 405. After the stream of polymer solution is stretched to its critical point, one or more streams of polymer solution emerges from nozzle 427 of nozzle assembly 405, and/or at a location below nozzle assembly 405, and move toward the negatively charged conduit 120. Using a volatile solvent, the solution dries substantially during transit and the fiber is deposited on conduit 120.

Mandrel 250 is configured to rotate about an axis, such as axis 435, with nozzle 427 typically oriented orthogonal to axis 435. The rotation around axis 435 allows the fiber matrix to be deposited along all sides, or around the entire circumference of conduit 120. In some embodiments, two motors 440a and 440b, as illustrated in Fig. 11, are used to rotate mandrel 250. Alternatively, electrospinning unit 400 can include a single motor configured to rotate mandrel 250. The rate of rotation of mandrel 250 can determine how the electrospun fibers are applied to one or more segments of conduit 120. For example, for a thicker portion of fiber matrix 110, the rotation rate can be slower than when a thinner portion of fiber matrix 110 is desired.

In addition to mandrel 250 rotating around axis 435, the nozzle assembly 405 can move, such as when driven by drive assembly 445 in a reciprocating or oscillating horizontal motion. Drive assemblies 445, as well as drive assembly 645 which operably attaches to fiber modifying assembly 605, can comprise a linear drive assembly, not shown, but typically a belt driven drive assembly comprising two or more pulleys driven by one or more stepper motors. Additionally or alternatively, assemblies 405 and/or 605 can be constructed and arranged to rotate around axis 435, rotating means not shown. The length of drive assemblies 445 and/or 645 and the linear motion applied to assemblies 405 and 605, respectively, can vary based on the length of conduit 120 to which a fiber matrix is delivered and/or a fiber matrix modification is applied. For example, the supported linear motion of drive assemblies 445 and/or 645 can be about 10cm to about 50cm. Assemblies 405 and/or 605 can move along the entire length or specific portions of the length of conduit 120. In some embodiments, fiber and/or modification is applied to the entire length of conduit 120 plus an additional 5cm (to mandrel 250) on either or both ends of conduit 340. In some embodiments, fiber(s) and/or modification is applied to the entire length of conduit 120 plus at least 1cm beyond either or both ends of conduit 120.

Assemblies 405 and/or 605 can be controlled such that specific portions along the length of conduit 120 are reinforced with a greater amount of fiber matrix as compared to other or remaining portions. Alternatively or additionally, assemblies 405 and/or 605 can be controlled such that specific portions of the length of conduit 120 include one or more kink resistant elements positioned at those one or more specific conduit 120 portions. In addition, conduit 120 can be rotating around axis 435 while assemblies 405 and/or 605 move, via drive assemblies 445 and/or 645, respectively, to position assemblies 405 and/or 605 at the particular portion of conduit 120 to which fiber is applied and/or modified. In some typical embodiments, assemblies 405 and/or 605 are translated back and forth at a velocity of approximately 200 mm/sec. Rotational speeds of mandrel 250 and translational speeds of assemblies 405 and/or 605 can be relatively constant, or can be varied during the process.

System 10 can also include a power supply, not shown, but configured to provide the electric potentials to nozzle 427 and mandrel 250, as well as to supply power to other components of system 10 such as drive assemblies 445 and 645 and fiber modification assembly 605. The power supply can be connected, either directly or indirectly, to at least one of mandrel 250 and conduit 120. Power can be transferred from the power supply to each component by, for example, one or more wires.

System 10 can also include inlet and/or outlet ports, not shown, but typically configured to control the environment surrounding the environment surrounding mandrel 250. A port can be configured to be both an inlet port and an outlet port. System 10 can include a housing, also not shown, but typically attachable to electrospinning unit 400 and defining a chamber surrounding assemblies 405 and/or 605 and/or mandrel 250, such that the ports can control a more limited (smaller) environment surrounding assemblies 405 and/or 605 and/or mandrel 250. Additionally or alternatively, the ports can be used to introduce or remove one or more gases, introduce or remove humidity, control temperature, control sterility, provide other environmental controls, and combinations of these.
introduce or remove humidity, control temperature, control sterility, provide other environmental controls, and combinations of these.

While the graft devices described herein have been described in detail as typically including a fiber matrix and a kink resisting element, other types of coverings can be used, such as a one or more fibrous or non-fibrous conformal structures circumferentially surrounding a tubular conduit. The conformal structures can be modified to include the kink resisting element, and/or a separate kink resisting element can be included in the graft device.

## Claims

1. A graft device for a mammalian patient, the graft device comprising:
a tubular conduit;
an electrospun fiber matrix surrounding the tubular conduit; and
a kink resisting element formed by laser modification to said electrospun fiber matrix to cause stiffening of a portion of said electrospun fiber matrix.

2. The graft device of claim 1, wherein the graft device further comprises an adhesive layer.

3. The graft device of claim 1, wherein the kink resisting element comprises a biodegradable material.

4. The graft device of claim 1, wherein the portion further comprises a modified hardness, a modified thickness, a modified shape, a modified material construction, or any combination thereof.

5. The graft device of claim 1, wherein the portion comprises a helical section of the fiber matrix.

## Patentansprüche

1. Transplantatvorrichtung für einen Säugerpatienten, wobei die Transplantatvorrichtung umfasst:
eine tubuläre Leitung;
eine elektrogesponnene Fasermatrix, die die tubuläre Leitung umgibt; und
ein knickbeständiges Element, das durch Lasermodifikation an der elektrogesponnenen Fasermatrix gebildet wird, um eine Versteifung eines Abschnitts der elektrogesponnenen Fasermatrix zu bewirken.

2. Transplantatvorrichtung nach Anspruch 1, wobei die Transplantatvorrichtung weiter eine Klebeschicht umfasst.

3. Transplantatvorrichtung nach Anspruch 1, wobei das knickbeständige Element ein biologisch abbaubares Material umfasst.

4. Transplantatvorrichtung nach Anspruch 1, wobei der Abschnitt weiter eine modifizierte Härte, eine modifizierte Dicke, eine modifizierte Form, eine modifizierte Materialkonstruktion oder eine beliebige Kombination davon umfasst.

5. Transplantatvorrichtung nach Anspruch 1, wobei der Abschnitt ein schraubenförmiges Teilstück der Fasermatrix umfasst.

## Revendications

1. Dispositif de greffe pour un patient mammifère, le dispositif de greffe comprenant:
un conduit tubulaire ;
une matrice de fibres électrofilées entourant le conduit tubulaire ; et
un élément résistant au vrillage formé par modification au laser sur ladite matrice de fibres électrofilées afin de provoquer le raidissement d'une partie de ladite matrice de fibres électrofilées.

2. Dispositif de greffe selon la revendication 1, dans lequel le dispositif de greffe comprend en outre une couche adhésive.

3. Dispositif de greffe selon la revendication 1, dans lequel l'élément résistant au vrillage comprend un matériau biodégradable.

4. Dispositif de greffe selon la revendication 1, dans lequel la partie comprend en outre une dureté modifiée, une épaisseur modifiée, une forme modifiée, une construction de matériau modifiée ou toute combinaison de celles-ci.

5. Dispositif de greffe selon la revendication 1, dans lequel la partie comprend une section hélicoïdale de la matrice de fibres.
